# FASCICULE DE BREVET EUROPEEN

(11) **EP 1 842 070 B1**
(45) Date de publication et mention de la délivrance du brevet: **13.04.2016**
(21) Numéro de dépôt: 06709119.9
(22) Date de dépôt: 18.01.2006
(51) Int. Cl.: G01N 33/66

(54) **METHODE D'ANALYSE D'OLIGOSACCHARIDES A PARTIR DE PLASMA SANGUIN**
VERFAHREN ZUR ANALYSE VON OLIGOSACCHARIDEN AUS BLUTPLASMA
METHOD FOR ANALYZING OLIGOSACCHARIDES FROM BLOOD PLASMA

(30) Priorité: 19.01.2005 FR 0500554
(43) Date de publication de la demande: 10.10.2007
(73) Titulaire: Aventis Pharma S.A., 92160 Antony (FR)
(72) Inventeur: MOURIER, Pierre, 94220 Charenton Le Pont (FR); VISKOV, Christian, 75013 Paris (FR)
(74) Mandataire: Jacobson, Claude
(86) Numéro de dépôt international: PCT/FR2006/000112
(87) Numéro de publication internationale: WO 2006/077317

(56) Documents cités:
- WO-A-00/13027
- WO-A-2004/027087
- US-A1- 2003 203 385

## Description

La présente invention a pour objet une méthode d'analyse des oligosaccharides constituant les héparines de bas poids moléculaire et les héparines de très bas poids moléculaire à partir du plasma sanguin.

Les héparines sont des agents biologiquement actifs de la famille des glycosaminoglycanes, et qui ont des propriétés anticoagulantes particulièrement utiles. Les héparines de bas poids moléculaire (HBPM) et les héparines de très bas poids moléculaire (HTBPM) sont préparées par coupure des chaînes polysaccharidiques longues d'héparine en chaînes plus courtes de bas poids moléculaire.

Les oligosaccharides constituant les HBPM et les HTBPM sont habituellement caractérisés par des paramètres macroscopiques tels que leur masse moléculaire moyenne ou des activités biologiques comme, par exemple, l'activité aXa (UI/mg). Celle-ci, qui mesure l'interaction avec l'antithrombine III, est la plus couramment utilisée.

Cependant, la mesure de ces paramètres macroscopiques ou de ces activités biologiques ne permet pas de répondre à plusieurs questions cruciales. En particulier, il est impossible de faire l'analyse des profils pharmacocinétiques des différents oligosaccharides constituant les HBPM et les HTBPM en se basant sur ces critères. Par exemple, l'activité aXa ne prend en compte que les espèces affines à l'AT III, c'est-à-dire environ 20 % du mélange seulement ; par conséquent, le comportement de 80% des oligosaccharides ne peut pas être mesuré. De plus, la contribution de chaque espèce constituant le mélange affin ne peut être déterminée.

Il est donc impossible de déterminer précisément la concentration plasmatique de chaque oligosaccharide et, à plus forte raison, son profil pharmacocinétique, par simple mesure de son activité aXa. Or ces profils apporteraient des informations très précieuses pour améliorer la posologie des médicaments à base de HBPM et de HTBPM.

Pour pouvoir analyser le comportement des différents oligosaccharides constituant une HBPM ou une HTBPM dans le plasma sanguin, il faut pouvoir séparer complètement et reproductiblement ces oligosaccharides des autres constituants du plasma, les protéines en particulier. Les méthodes décrites dans l'art antérieur font appel à des traitements protéasiques soutenus (Volpi et al.,1995, Biochim. Biophys. Acta., 1243 (1) : 49-58).

Des méthodes de chromatographie ou d'électrophorèse permettent l'analyse des différents constituants des HBPM. Par exemple, l'électrophorèse capillaire (EC) est utilisée pour la séparation des oligosaccharides d'héparine (Guerrini et al., Glycobiology, 2002, 12 : 713-719 ; Linhardt et al., BioMethods, 1997, 9 : 183-197). Cependant, la sélectivité de l'EC est plutôt faible par rapport à la chromatographie liquide. Il est aussi possible, dans certains cas, d'utiliser la spectrophotométrie de masse MALDI-TOF pour l'analyse des oligosaccharides hépariniques, mais elle ne peut s'appliquer aux mélanges complexes, sans même mentionner son prix élevé.

De ce fait, l'analyse des oligosaccharides sulfatés, comme ceux constituant les HBPM et les HTBPM se fait surtout par chromatographie liquide haute performance (CLHP). Il a récemment été décrit une méthode d'analyse des HBPM consistant en une dépolymérisation enzymatique suivie d'une réduction le cas échéant, et d'un dosage par CLHP (WO 2004/027087). Une demande européenne récente (EP04290789.9) divulgue une méthode de dosage des oligosaccharides constituant les HBPM et les HTBPM par chromatographie d'échange d'anions CTA-SAX. Cette méthode fait appel à une chromatographie échangeuse d'anions conduite sur un sel d'ammonium quaternaire, en particulier le cétyl méthyl ammonium, adsorbé dynamiquement sur une colonne de silice de phase inverse et qui maintient une charge positive nette et constante dans une gamme de pH comprise entre 2 et 12. Cette méthode peut impliquer un traitement préalable, dépolymérisation ou séparation par chromatographie d'exclusion, avant l'analyse des HBPM et des HTBPM par CLHP sur colonne CTA-SAX.

US2003/0203385 décrit une méthode d'analyse de polysaccharides sulfatés au moyen de diverses méthodes d'analyse, dont l'électrophorèse capillaire, mais ne décrit pas de filtration ni d'analyse par HPLC.
La présente invention a pour objet une méthode d'analyse des oligosaccharides constituants les héparines de bas poids moléculaire et les héparines de très bas poids moléculaire à partir du plasma sanguin, caractérisée en ce que l'on effectue les deux étapes suivantes :
- un traitement de l'échantillon de plasma sanguin par filtration,
- un dosage par CLHP ;
et en ce qu'elle comporte une méthode de détection permettant de détecter sélectivement les sucres acétylés, ladite méthode de détection s'effectuant en prenant comme signal la différence entre l'absorbance à deux longueurs d'onde choisies de telle façon que l'absorptivité des saccharides non acétylés s'annule.

Cette méthode n'implique ni dépolymérisation enzymatique ni fractionnement par chromatographie d'exclusion des oligosaccharides présents dans l'échantillon de plasma sanguin avant le dosage par CLHP. Par contre, selon cette méthode, l'échantillon de plasma sanguin est traité par filtration de façon à séparer les oligosaccharides, que l'on retrouve dans le filtrat, des contaminants plasmatiques, en particulier des protéines, qui restent dans le concentrat.

L'invention a donc plus particulièrement pour objet une méthode telle que définie plus haut où l'échantillon de plasma sanguin est traité par filtration. L'invention a tout particulièrement pour objet une méthode telle que définie plus haut caractérisée en ce que les molécules dont le poids moléculaire est supérieur à 30 kDa sont retenues dans le concentrat.

Pour permettre une quantification de la méthode de traitement de l'échantillon de plasma sanguin et vérifier la reproductibilité de ce traitement d'un échantillon à l'autre, on ajoute, le cas échéant, un étalon interne à l'échantillon. Cet étalon doit être un oligosaccharide, si possible proche structurellement du produit dosé, qui est mélangé au plasma juste avant la filtration.

L'invention a donc aussi pour objet une méthode telle que définie plus haut caractérisée en ce qu'un oligosaccharide témoin est ajouté à l'échantillon de plasma juste avant la filtration. l'autre, on ajoute, le cas échéant, un étalon interne à l'échantillon. Cet étalon doit être un oligosaccharide, si possible proche structurellement du produit dosé, qui est mélangé au plasma juste avant la filtration.

L'invention a donc aussi pour objet une méthode telle que définie plus haut caractérisée en ce qu'un oligosaccharide témoin est ajouté à l'échantillon de plasma juste avant la filtration.

Bien que la plupart des oligosaccharides soit séparée des protéines par la filtration, il est possible qu'une partie reste piégée par des interactions électrostatiques avec le concentrat. Dans ce cas, il est nécessaire de faire un ou plusieurs lavages du concentrat dans une solution saline.

L'invention a donc aussi pour objet une méthode telle que définie plus haut et caractérisée en ce que le concentrat est lavé au moins une fois dans une solution saline.

Selon l'invention, la solution saline est de préférence une solution de chlorure de potassium.

L'invention a plus particulièrement pour objet une méthode telle que définie plus haut caractérisée en ce que la concentration de la solution saline après addition au concentrat est supérieure ou égale à 1M, et tout particulièrement une telle méthode où la concentration saline est supérieure ou égale à 2 M.

Après un ou plusieurs lavages avec une solution saline, le concentrat est lavé à l'eau, le cas échéant. L'invention a donc pour objet une méthode telle que définie plus haut comprenant après un lavage au moins du concentrat dans une solution saline, une étape de lavage à l'eau.

Le filtrat originel est rassemblé avec les filtrats provenant des lavages et l'ensemble est dilué 5 fois dans l'eau. Cette dilution permet de reconcentrer la totalité des oligosaccharides après l'injection sur la colonne chromatographique, tout en évitant tout élargissement des pics causé par une concentration saline trop élevée. Juste avant l'injection, le pH de la solution est ajusté à 3 par addition de HCl 1 N.

Selon la méthode selon l'invention, la totalité du filtrat et, le cas échant, des lavages est injectée directement et analysée par CLHP.

La CLHP échangeuse d'anions est la méthode séparative la mieux adaptée à un tel mélange complexe. En particulier, on utilise pour la CLHP une colonne d'échange d'anions avec greffage de l'ammonium quaternaire par liaison covalente. Ce type de colonne offre l'avantage de pouvoir utiliser comme phase mobile le perchlorate, ce qui est impossible avec une colonne CTA-SAX à cause de la forte complexation entre perchlorate et CTA.

L'invention a donc aussi pour objet une méthode telle que définie plus haut où les oligosaccharides sont dosés par CLHP échangeuse d'anions. Elle a plus particulièrement pour objet une méthode telle que définie plus haut où les oligosaccharides sont dosés par CLHP sur colonne d'échange d'anions avec greffage de l'ammonium quaternaire par liaison covalente.

Les colonnes de type IonPAc AS11 (Dionex), de granulométrie 9-13 µm et de longueur 25 cm, peuvent être utilisées. Tous les diamètres de colonnes classiques, compris entre 1 mm et 4,6 mm sont utilisables, mais on utilise de préférence des colonnes de diamètre de 2 mm. Dans un cadre plus général, les colonnes de diamètre plus faible, par exemple 1mm, peuvent être utilisées, nécessitant de fait une quantité de plasma plus faible, et permettant également un gain en sensibilité de l'analyse.

L'appareillage utilisé peut être un chromatographe permettant la formation de gradient d'élution avec un détecteur UV. On utilisera de préférence un détecteur muni d'une barrette de diodes, qui permet de réaliser des spectres UV des constituants et d'enregistrer des signaux complexes, résultant de la différence entre les absorbances à 2 longueurs d'onde différentes et permettant la détection des oligosaccharides acétylés parmi les composés d'origine plasmatique. L'utilisation d'une phase mobile transparente jusqu'à 200 nm permet de faciliter la différenciation des constituants. La phase mobile utilisée ici sera de préférence une solution de perchlorate de sodium, mais les sels de méthane sulfonate ou phosphate peuvent aussi être utilisés.

L'invention a donc pour objet une méthode telle que définie plus haut caractérisée en ce qu'on utilise une phase mobile transparente jusqu'à 200 nm. En particulier, l'invention a pour objet une méthode telle que définie plus haut caractérisée en ce que la phase mobile est une solution de perchlorate de sodium, de méthane sulfonate ou de phosphate. L'invention a tout particulièrement pour objet une méthode telle que définie plus haut caractérisée en ce que la phase mobile est une solution de perchlorate de sodium.

Le pH préconisé pour la séparation est compris entre 2 et 6,5. Préférentiellement, on utilisera un pH voisin de 3. Ce pH sera obtenu par addition d'un sel tel que le phosphate possédant un pouvoir tampon à pH = 3 meilleur que celui des perchlorates.

A titre d'exemple, on donne ci-après des conditions standards de séparation chromatographique :
- Solvant A : NaH₂PO₄ 2,5 mM porté à pH 2,9 par addition de H₃PO₄
- Solvant B : NaClO₄ 1N - NaH₂PO₄ 2,5 mM porté à pH 3,0 par addition de H₃PO₄
Le gradient d'élution peut être le suivant :
T = 0 min : %B = 3 ; T = 40 min: %B = 60 ; T = 60 min : %B = 80
Pour une colonne de 2 mm de diamètre, le débit choisi sera, par exemple, de 0,22 ml/min et la température de la colonne de 40°C.

Les oligosaccharides présents dans les échantillons de plasma filtré sont détectés par UV. Comme les polysaccharides non acétylés ont tous, à un pH donné, un spectre UV presque identique, il est possible de détecter sélectivement les sucres acétylés en prenant comme signal la différence entre les absorbances à deux longueurs d'onde choisies de telle façon que l'absorptivité des saccharides non acétylés s'annule.

Dans le cas ci-dessous, on choisira 202 nm et 230 nm comme longueurs d'onde de référence et on notera le signal 202 - 230 nm. Il est évident pour l'homme du métier que le choix de la longueur d'onde sera dépendant du pH de la phase mobile, des ajustements de quelques nm pouvant être nécessaires pour être à l'optimum des dites conditions. Le détecteur le plus adapté à cette technique est le détecteur DAD de la société Agilent Technologies. Dans ce cas, on réalise une double détection sur chaque échantillon, à 234 nm d'une part et, d'autre part, à 202 - 230 nm. On peut également réaliser une triple détection, en mesurant en sus l'absorbance de chaque échantillon à 280 nm, ce qui permet de vérifier que le signal n'est pas contaminé par des résidus protéiques.

La présente invention a donc pour objet une méthode telle que définie plus haut caractérisée en ce que la méthode de détection permet de détecter sélectivement les sucres acétylés.

La présente invention a aussi pour objet une méthode telle que définie plus haut caractérisée en ce que la méthode de détection sélective des sucres acétylés s'effectue en prenant comme signal la différence entre l'absorbance à deux longueurs d'onde choisies de telle façon que l'absorptivité des saccharides non acétylés s'annule.

La présente invention a donc tout particulièrement pour objet une méthode telle que définie plus haut caractérisée en ce que la méthode de détection sélective des sucres acétylés s'effectue en mesurant l'absorbance à 202 - 230 nm et 234 nm.

La présente invention a donc tout particulièrement pour objet une méthode telle que définie plus haut caractérisée en ce que la méthode de détection sélective des sucres acétylés s'effectue en mesurant l'absorbance à 202 - 230 nm, 234 nm et 280 nm.

La méthode définie plus haut permet plus particulièrement d'analyser tous les oligosaccharides β-insaturés. Plus particulièrement, elle s'applique aux oligosaccharides constituant l'enoxaparine, l'octaparine, la bemiparine et la tinzaparine.

### Exemple expérimental

### Mode opératoire

### Traitement du plasma

Un échantillon de plasma de volume compris entre 100 et 1000 µl est prélevé et, le cas échéant, l'étalon interne est ajouté à celui-ci.
Après homogénéisation, la solution résultante est introduite dans une cartouche d'ultrafiltration Ultrafree05 de 30KD (Millipore) préalablement rincée par 250 µl d'eau par centrifugation 5 minutes à 10000 T/min. Le plasma est introduit dans la partie supérieure de la cartouche en 1 ou 2 fois si les quantités à filtrer sont supérieures à 500 µl.
La cartouche est centrifugée à 10000 T/min entre 1 et 2 heures, puis le filtrat est prélevé. Le cas échéant, la deuxième partie du plasma à filtrer est introduite dans la partie supérieure de la cartouche et celle-ci est centrifugée de nouveau. La quantité exacte de plasma introduite aura été pesée. La cartouche est centrifugée jusqu'à ce que le résidu protéique dans la partie supérieure de la cartouche ne représente plus qu'environ 10% de la partie initiale.
Le filtrat est prélevé et on ajoute au concentrat 210 µl de KCl 3M par ml de plasma filtré.
Après homogénéisation, la cartouche est centrifugée à 10000 T/min entre 1 et 4 heures. La cartouche est centrifugée jusqu'à ce que le résidu protéique dans la partie supérieure de la cartouche ne représente plus qu'environ 10% de la partie initiale.
Le deuxième filtrat est prélevé et on ajoute une deuxième fois au concentrat 210 µl de KCl 3M par ml de plasma filtré. Après homogénéisation, la cartouche est centrifugée à 10000 T/min entre 1 et 4 heures. La cartouche est centrifugée jusqu'à ce que le résidu protéique dans la partie supérieure de la cartouche ne représente plus qu'environ 10% de la partie initiale.

Le troisième filtrat est prélevé et on ajoute 250 µl d'eau par ml de plasma filtré.
Après homogénéisation, la cartouche est centrifugée à 10000 T/min entre 1 et 4 heures. La cartouche est centrifugée jusqu'à ce que le résidu protéique dans la partie supérieure de la cartouche ne représente plus qu'environ 10% de la partie initiale.
Les filtrats sont rassemblés, dilués 5 fois dans l'eau et la totalité est injectée directement en CLHP d'échange d'anions. Juste avant l'injection, le pH de la solution injectée est ajusté à 3 par addition de HCl 1N.

### Méthode CLHP

Le plasma filtré est analysé par CLHP d'échange d'ions. Les colonnes utilisées sont des colonnes IonPAc As11 (Dionex), de longueur 25 cm et de diamètre intérieur de 2 mm. Le détecteur utilisé est un spectrophotomètre UV muni d'une barrette de diodes.
La totalité du pH est injecté après ajustement du pH à 3 par addition de HCl N. Le débit choisi est de 0,22 ml/min ; la température de la colonne est de 40°C.
L'élution est réalisée en mode gradient. Les deux solutions utilisées sont :
Solvant A : NaH₂PO₄ 2,5 mM, porté à pH 2,9 par addition de H₃PO₄.
Solvant B : NaClO₄ 1 N, NaH₂PO₄ 2,5mM, porté à pH 3,0 par addition de H₃PO₄.
Le gradient d'élution est le suivant :
T = 0 min : % B = 3 ; T = 40 min: % B = 60 ; T = 60 min : % B = 80

### Exemple 1

On suit le devenir d'un mélange de trois octasaccharides dans le sang de chiens mâles beagle. Ces 3 octasaccharides, symbolisés ΔIIaIIsIsIs, ΔIsIIaIIsIs et ΔIIaIIsIsIs¹, ^{6-anhydro} sont les principaux octasaccharides affins des fractions octasaccharides du Lovenox®. Ils possèdent une forte affinité à l'ATIII et par conséquent une activité anti-thrombotique.

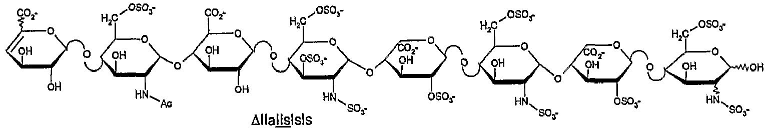

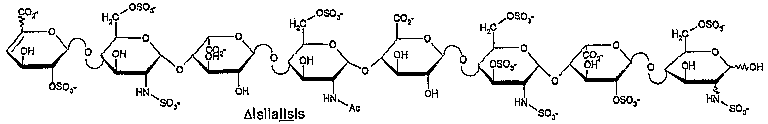

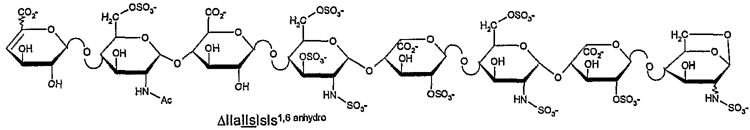

Le mélange est administré par voie sous cutanée à 3 chiens mâles beagle, à une dose cible sur chacun de ces 3 constituants de 0,5 mg/kg. La solution d'administration est une solution à 0,9 % NaCl du mélange des 3 octasaccharides à la concentration cible de 0,5 mg/ml.
Des prélèvements sanguins de 5 ml sont réalisés aux temps 0 ; 0,25 h ; 0,5 h ; 1 h ; 2 h ; 4 h ; 6 h ; 8 h ; 24 h ; 30 h et 48 h après l'administration. Les prélèvements sanguins sont collectés dans des tubes contenant 400 µl d'un mélange CTAD (citrate, théophylline, adénosine, dipyridamole). Après homogénéisation, la solution est centrifugée à 3000 g pendant 15 min à 15°C. Le surnageant plasmatique est récupéré et conservé à -20°C jusqu'à usage.
Pour le traitement du plasma avant injection CLHP, environ 1 ml de plasma est prélevé et pesé, auquel on additionne 50 µl d'une solution de l'étalon interne à une concentration d'environ 0,02 g/l.

On choisit l'étalon interne de façon à ce qu'il soit structurellement le plus proche possible des produits à analyser. Sa structure est donnée ci-dessous.

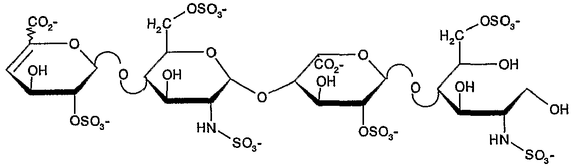

Le tétrasaccharide utilisé permet de corriger l'influence potentielle des variations du rendement d'extraction. Le plasma est traité et analysé par CLHP.

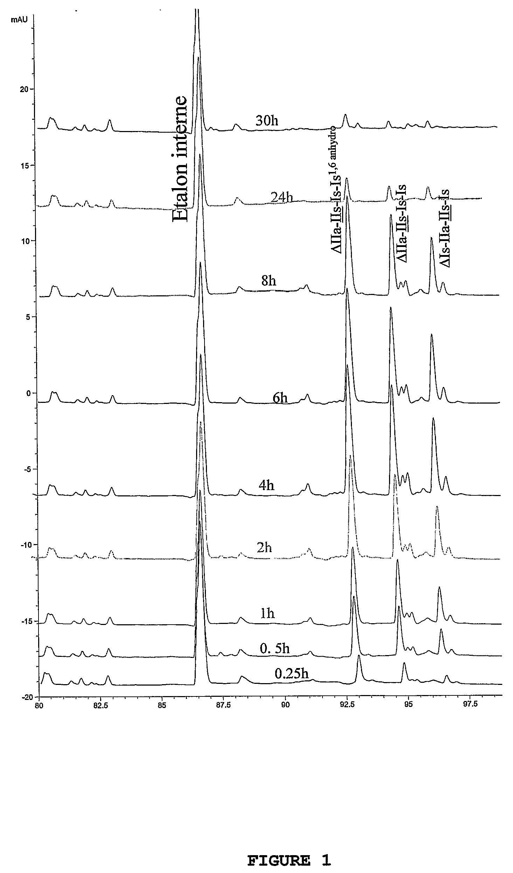

Les résultats obtenus sont représentés dans le Tableau 1. Les chromatogrammes correspondants sont représentés sur la Figure 1.

**Tableau 1 : Suivi de la concentration plasmatique (µM) des 3 octasaccharides en fonction du temps après administration**

| | ΔIIaIIsIsIs1,6 anhydro : Concentration en µMole/l | | |
|---|---|---|---|
| Temps (heures) | Chien1 | Chien2 | Chien3 |
| 0 | 0,00 | 0,00 | 0,00 |
| 0,25 | 0,05 | 0,05 | 0,06 |
| 0,5 | 0,17 | 0,10 | 0,17 |
| 1 | 0,34 | 0,21 | 0,24 |
| 2 | 0,56 | 0,32 | 0,34 |
| 4 | 0,44 | 0,30 | 0,46 |
| 6 | 0,37 | 0,37 | 0,38 |
| 8 | 0,30 | 0,25 | 0,30 |
| 24 | 0,06 | 0,05 | 0,05 |
| 30 | 0,03 | 0,03 | 0,04 |
| 48 | 0,01 | 0,01 | 0,01 |
| | | | |

| | ΔIIaIIsIsIs: Concentration en µMole/l | | |
|---|---|---|---|
| Temps (heures) | Chien1 | Chien2 | Chien3 |
| 0 | 0,00 | 0,00 | 0,00 |
| 0,25 | 0,04 | 0,05 | 0,06 |
| 0,5 | 0,18 | 0,10 | 0,17 |
| 1 | 0,33 | 0,20 | 0,24 |
| 2 | 0,59 | 0,32 | 0,33 |
| 4 | 0,47 | 0,30 | 0,45 |
| 6 | 0,36 | 0,37 | 0,36 |
| 8 | 0,28 | 0,26 | 0,29 |
| 24 | 0,04 | 0,04 | 0,04 |
| 30 | 0,02 | 0,01 | 0,02 |
| 48 | 0,00 | 0,00 | 0,00 |

| | ΔIsIIaIIsIs: Concentration en µMole/l | | |
|---|---|---|---|
| Temps (heures) | Chien1 | Chien2 | Chien3 |
| 0 | 0,00 | 0,00 | 0,00 |
| 0,25 | 0,01 | 0,03 | 0,02 |
| 0,5 | 0,07 | 0,06 | 0,09 |
| 1 | 0,16 | 0,11 | 0,13 |
| 2 | 0,33 | 0,20 | 0,19 |
| 4 | 0,27 | 0,18 | 0,28 |
| 6 | 0,22 | 0,23 | 0,23 |
| 8 | 0,18 | 0,15 | 0,19 |
| 24 | 0,04 | 0,03 | 0,03 |
| 30 | 0,02 | 0,02 | 0,02 |
| 48 | 0,00 | 0,00 | 0,00 |

### Exemple 2

Une HTBPM de masse moléculaire moyenne 2500 Da est administrée à la dose cible de 1,4 mg/kg à des volontaires humains. Des prélèvements sanguins sont réalisés aux temps 0 ; 0,25 h ; 0,5 h ; 0,75 h ; 1 h ; 1,25 h ; 1,5h ; 2 h ; 2,5h ; 3 h ; 3,5 h ; 4 h ; 4,5 h ; 5 h ; 6 h ; 7 h ; 8 h ; 9 h ; 10 h ; 12 h ; 14 h et 24 h après l'administration.
La HTBPM utilisée étant un mélange complexe d'oligosaccharides, l'étude a été limitée à certains éléments choisis de ce mélange.
D'autre part, comme il pas été trouvé d'étalon interne qui n'interfère pas au niveau chromatographique avec un des constituants du mélange, le dosage a été fait en prenant comme coefficient de réponse molaire celui obtenu dans l'exemple 1 sur les octasaccharides affins.

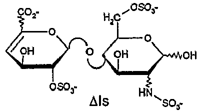

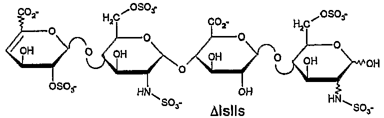

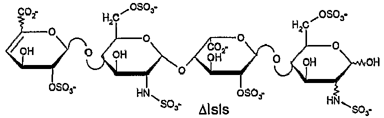

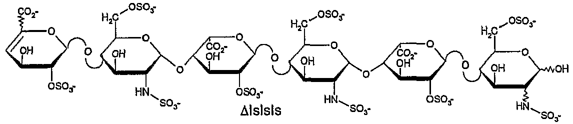

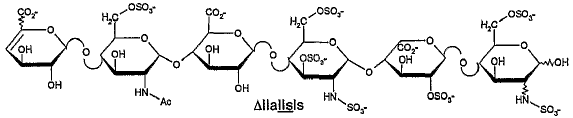

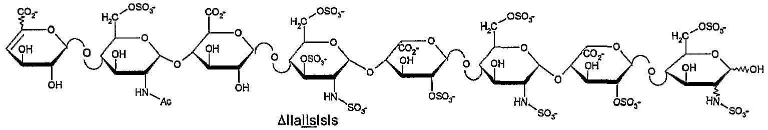

Les résultats mettent en évidence une très grande différence de comportement parmi les oligosaccharides constituant l'héparine de bas poids moléculaire étudiée. Les oligosaccharides ayant une activité AXa ont une cinétique d'élimination beaucoup plus lente que les autres oligosaccharides sulfatés, ce qui est indécelable à partir d'un suivi d'activité AXa.

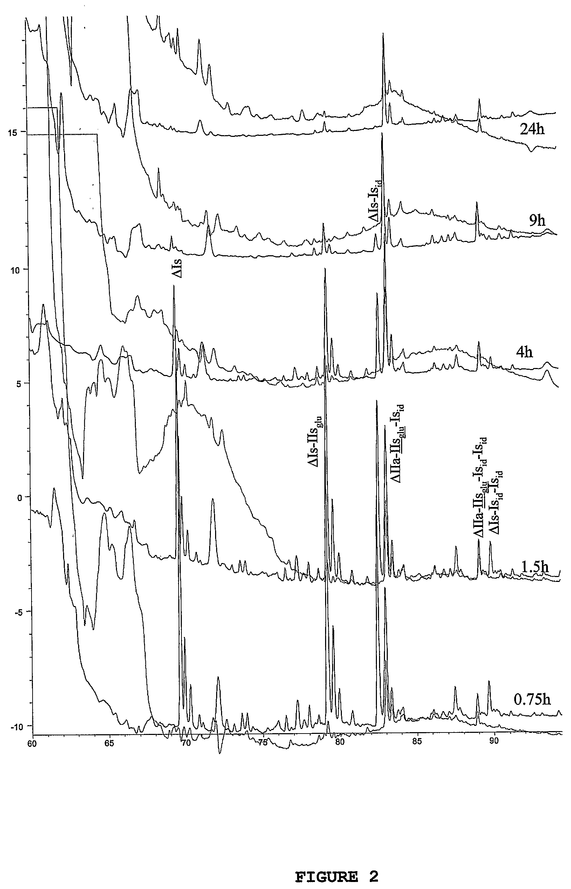

Les résultats obtenus sont représentés dans le Tableau 2. Les chromatogrammes correspondants sont représentés sur la Figure 2.

**Tableau 2 : Suivi de la concentration plasmatique (µM) des principaux oligosaccharides en fonction du temps après administration**

| Temps en heures | ΔIs | ΔIsIIs | ΔIsIs | ΔIIaIIsIs | ΔIsIsIs | ΔIIaIIsIsIs |
|---|---|---|---|---|---|---|
| 0 | 0,00 | 0,00 | 0,00 | 0,00 | 0,00 | 0,00 |
| 0,25 | 0,24 | 0,20 | 0,16 | 0,05 | 0,02 | 0,01 |
| 0,5 | 0,30 | 0,30 | 0,24 | 0,10 | 0,04 | 0,03 |
| 0,75 | 0,26 | 0,26 | 0,21 | 0,10 | 0,03 | 0,02 |
| 1 | 0,23 | 0,26 | 0,20 | 0,10 | 0,04 | 0,03 |
| 1,25 | 0,20 | 0,23 | 0,19 | 0,12 | 0,04 | 0,04 |
| 1,5 | 0,19 | 0,22 | 0,19 | 0,11 | 0,03 | 0,04 |
| 2 | 0,16 | 0,18 | 0,15 | 0,11 | 0,03 | 0,04 |
| 2,5 | 0,11 | 0,13 | 0,10 | 0,09 | 0,02 | 0,03 |
| 3 | 0,10 | 0,10 | 0,09 | 0,08 | 0,02 | 0,03 |
| 3,5 | 0,08 | 0,10 | 0,07 | 0,09 | 0,02 | 0,04 |
| 4 | 0,07 | 0,07 | 0,07 | 0,10 | 0,01 | 0,02 |
| 4,5 | 0,06 | 0,06 | 0,06 | 0,10 | 0,01 | 0,04 |
| 5 | 0,04 | 0,05 | 0,05 | 0,09 | 0,01 | 0,03 |
| 6 | 0,03 | 0,04 | 0,04 | 0,08 | 0,01 | 0,02 |
| 7 | 0,03 | 0,03 | 0,03 | 0,10 | 0,01 | 0,04 |
| 8 | 0,02 | 0,02 | 0,02 | 0,07 | 0,00 | 0,02 |
| 9 | 0,01 | 0,01 | 0,01 | 0,07 | 0,00 | 0,02 |
| 10 | 0,01 | 0,01 | 0,01 | 0,07 | 0,00 | 0,02 |
| 12 | 0,00 | 0,00 | 0,01 | 0,08 | 0,00 | 0,03 |
| 14 | 0,00 | 0,00 | 0,00 | 0,07 | 0,00 | 0,02 |
| 24 | | | | 0,05 | | 0,01 |

Figure 1 :
   Suivi chromatographique de la pharmacocinétique de 3 octasaccharides pour le chien 1.
Figure 2 :
   Suivi chromatographique des principaux oligosaccharides constituant une héparine de très bas poids moléculaire en fonction du temps après administration.

## Revendications

1. Méthode d'analyse d'oligosaccharides constituant les héparines de bas poids moléculaire et les héparines de très bas poids moléculaire à partir de plasma sanguin, **caractérisée en ce que** l'on effectue les deux étapes suivantes :
a. un traitement de l'échantillon par filtration,
b. un dosage par Chromatographie Liquide Haute Performance (CLHP) ;
et **en ce qu'**elle comporte une méthode de détection permettant de détecter sélectivement les sucres acétylés,
ladite méthode de détection s'effectuant en prenant comme signal la différence entre l'absorbance à deux longueurs d'onde choisies de telle façon que l'absorptivité des saccharides non acétylés s'annule.

2. Méthode telle que définie à la revendication 1, **caractérisée en ce que** les molécules dont le poids moléculaire est supérieur à 30 kDa sont retenues dans le concentrat.

3. Méthode telle que définie à la revendication 2, **caractérisée en ce qu'**un oligosaccharide témoin est ajouté au plasma avant filtration.

4. Méthode telle que définie à la revendication 3, **caractérisée en ce que** le concentrat est lavé au moins une fois dans une solution saline.

5. Méthode telle que définie à la revendication 4, **caractérisée en ce que** la solution saline est une solution de chlorure de potassium.

6. Méthode telle que définie à l'une quelconque des revendications 4 à 5, **caractérisée en ce que** la concentration de la solution saline après addition du concentrat est supérieure ou égale à 1 M.

7. Méthode telle que définie à l'une quelconque des revendications 4 à 5, **caractérisée en ce que** la concentration de la solution saline après addition du concentrat est égale ou supérieure à 2 M.

8. Méthode telle que définie à la revendication 1, **caractérisée en ce que** le concentrat est lavé au moins une fois dans une solution saline puis lavé à l'eau.

9. Méthode telle que définie à l'une quelconque des revendications 1 à 8, **caractérisée en ce que** la totalité du filtrat et, le cas échant, des lavages est injectée et analysée par CLHP.

10. Méthode telle que définie à la revendication 9, **caractérisée en ce que** la méthode chromatographique est une chromatographie par échange d'anion.

11. Méthode telle que définie à la revendication 9, **caractérisée en ce que** la méthode chromatographique est une chromatographie par échange d'anion sur colonne d'échange d'anions avec greffage de l'ammonium quaternaire par liaison covalente.

12. Méthode telle que définie à la revendication 10, **caractérisée en ce qu'**on utilise une phase mobile transparente jusqu'à 200 nm.

13. Méthode telle que définie à la revendication 12, **caractérisée en ce que** la phase mobile est une solution de perchlorate de sodium, de méthane sulfonate ou de phosphate.

14. Méthode telle que définie à la revendication 13, **caractérisée en ce que** la phase mobile est une solution de perchlorate de sodium.

15. Méthode telle que définie à la revendication 1, **caractérisée en ce que** la méthode de détection sélective des sucres acétylés s'effectue en mesurant l'absorbance à 202 - 230 nm et 234 nm.

16. Méthode telle que définie à la revendication 15, **caractérisée en ce que** la méthode de détection sélective des sucres acétylés s'effectue en mesurant l'absorbance à 202 - 230 nm, 234 nm et 280 nm.

17. Application de la méthode telle que définie à la revendication 1 au dosage des oligosaccharides β-insaturés.

18. Application de la méthode telle que définie à la revendication 1 au dosage de l'enoxaparine, l'octaparine, la bemiparine ou la tinzaparine.

19. Application de la méthode telle que définie à la revendication 1 au dosage de l'un ou plusieurs des octasaccharides suivants :

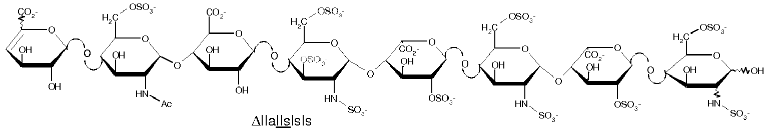

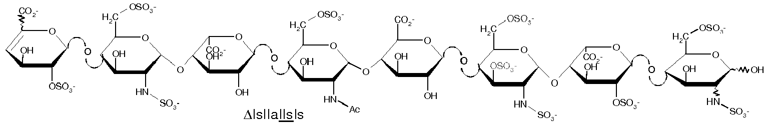

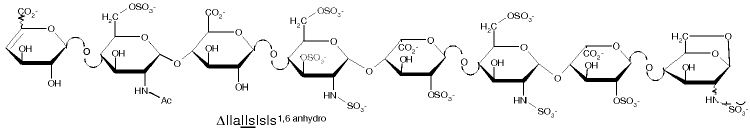

20. Application de la méthode telle que définie à la revendication 1 au dosage de l'un ou plusieurs des oligosaccharides suivants :

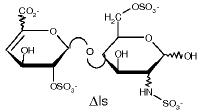

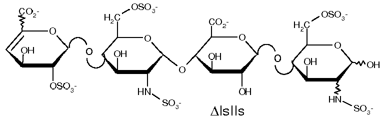

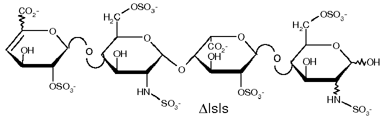

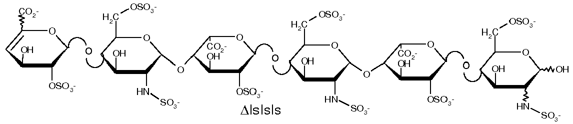

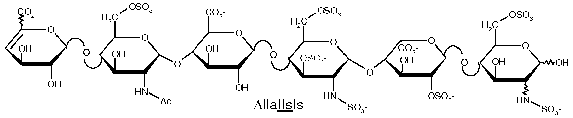

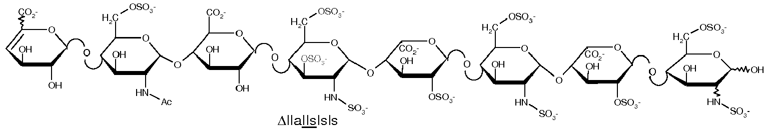

## Patentansprüche

1. Verfahren zur Analyse von Oligosacchariden, die aus Heparinen mit niedrigem Molekulargewicht und Heparinen mit sehr niedrigem Molekulargewicht bestehen, ausgehend von Blutplasma, **dadurch gekennzeichnet, dass** man die beiden folgenden Schritte durchführt:
a. Behandlung der Probe durch Filtration,
b. quantitative Bestimmung durch HochdruckFlüssigkeitschromatographie (HPLC);
und dass es ein Nachweisverfahren, das den selektiven Nachweis von acetylierten Zuckern erlaubt, umfasst,
wobei das Nachweisverfahren dadurch durchgeführt wird, dass als Signal die Differenz zwischen die Absorbanz bei zwei Wellenlängen, die so gewählt werden, dass das Absorptionsvermögen der nicht acetylierten Saccharide sich aufhebt, genommen wird.

2. Verfahren nach Anspruch 1, **dadurch gekennzeichnet, dass** die Moleküle mit einem Molekulargewicht von mehr als 30 kDa in dem Konzentrat zurückgehalten werden.

3. Verfahren nach Anspruch 2, **dadurch gekennzeichnet, dass** vor der Filtration ein Referenz-Oligosaccharid zu dem Plasma gegeben wird.

4. Verfahren nach Anspruch 3, **dadurch gekennzeichnet, dass** das Konzentrat mindestens einmal in einer Salzlösung gewaschen wird.

5. Verfahren nach Anspruch 4, **dadurch gekennzeichnet, dass** es sich bei der Salzlösung um eine Kaliumchloridlösung handelt.

6. Verfahren nach einem der Ansprüche 4 bis 5, **dadurch gekennzeichnet, dass** die Konzentration der Salzlösung nach der Zugabe des Konzentrats größer gleich 1 M ist.

7. Verfahren nach einem der Ansprüche 4 bis 5, **dadurch gekennzeichnet, dass** die Konzentration der Salzlösung nach der Zugabe des Konzentrats größer gleich 2 M ist.

8. Verfahren nach Anspruch 1, **dadurch gekennzeichnet, dass** das Konzentrat mindestens einmal in einer Salzlösung und dann mit Wasser gewaschen wird.

9. Verfahren nach einem der Ansprüche 1 bis 8, **dadurch gekennzeichnet, dass** die Gesamtheit des Filtrats und gegebenenfalls der Waschlösungen eingespritzt und durch HPLC analysiert wird.

10. Verfahren nach Anspruch 9, **dadurch gekennzeichnet, dass** es sich bei dem Chromatographieverfahren um eine Anionenaustauschchromatographie handelt.

11. Verfahren nach Anspruch 9, **dadurch gekennzeichnet, dass** es sich bei dem Chromatographieverfahren um eine Anionenaustauschchromatographie an einer Anionenaustauschersäule mit Aufpfropfung von quaternärem Ammonium über eine kovalente Bindung handelt.

12. Verfahren nach Anspruch 10, **dadurch gekennzeichnet, dass** man eine bis 200 nm transparente mobile Phase verwendet.

13. Verfahren nach Anspruch 12, **dadurch gekennzeichnet, dass** die mobile Phase zu eine Natriumperchlorat-, Methansulfonat- oder Phosphatlösung gehört.

14. Verfahren nach Anspruch 13, **dadurch gekennzeichnet, dass** die mobile Phase zu eine Natriumperchloratlösung gehört.

15. Verfahren nach Anspruch 1, **dadurch gekennzeichnet, dass** das Verfahren zum selektiven Nachweis von acetylierten Zuckern, durch Messen die Absorbanz bei 202 - 230 nm und 234 nm, durchgeführt wird.

16. Verfahren nach Anspruch 15, **dadurch gekennzeichnet, dass** das Verfahren zum selektiven Nachweis von acetylierten Zuckern, durch Messen die Absorbanz bei 202 - 230 nm, 234 nm und 280 nm, durchgeführt wird

17. Anwendung des Verfahrens nach Anspruch 1 zur quantitativen Bestimmung von β-ungesättigten Oligosacchariden.

18. Anwendung des Verfahrens nach Anspruch 1 zur quantitativen Bestimmung von Enoxaparin, Octaparin, Bemiparin oder Tinzaparin.

19. Anwendung des Verfahrens nach Anspruch 1 zur quantitativen Bestimmung eines oder mehrerer der folgenden Octasaccharide:

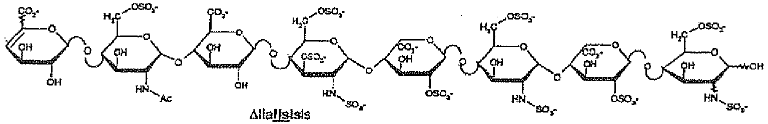

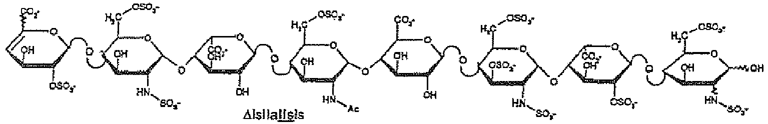

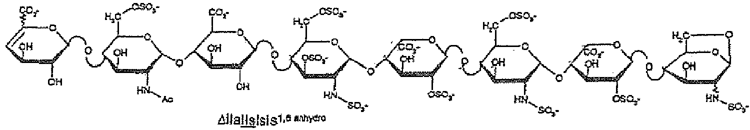

20. Anwendung des Verfahrens nach Anspruch 1 zur quantitativen Bestimmung eines oder mehrerer der folgenden Oligosaccharide:

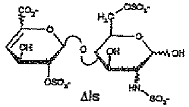

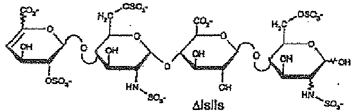

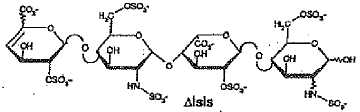

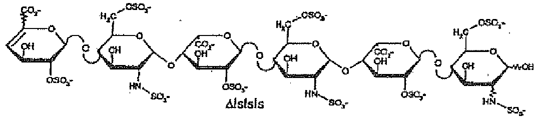

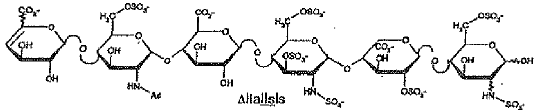

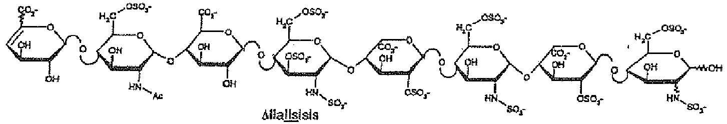

## Claims

1. Method for the analysis of oligosaccharides constituting low molecular weight heparin and very low molecular weight heparin from blood plasma, **characterized in that** the following two stages are carried out:
a. treatment of the sample by filtration,
b. assay by high performance liquid chromatography (HPLC); and **in that** it comprises a detection method allowing to selectively detect acetylated sugars, the said detection method being carried out by taking, as signal, the difference between the absorption at two wavelengths chosen in such a way that the absorptivity of the nonacetylated saccharides is cancelled out.

2. Method as defined in Claim 1, **characterized in that** the molecules which molecular weight greater than 30 kDa are retained in the concentrate.

3. Method as defined in Claim 2, **characterized in that** a control oligosaccharide is added to the plasma before filtration.

4. Method as defined in Claim 3, **characterized in that** the concentrate is washed at least once in a saline solution.

5. Method as defined in Claim 4, **characterized in that** the saline solution is a potassium chloride solution.

6. Method as defined in either one of Claims 4 and 5, **characterized in that** the concentration of the saline solution after addition of the concentrate is greater than or equal to 1 M.

7. Method as defined in either one of Claims 4 and 5, **characterized in that** the concentration of the saline solution after addition of the concentrate is greater than or equal to 2M.

8. Method as defined in Claim 1, **characterized in that** the concentrate is washed at least once in a saline solution and then washed with water.

9. Method as defined in any one of Claims 1 to 8, **characterized in that** the whole of the filtrate and, if appropriate, of the washings is injected and analyzed by HPLC.

10. Method as defined in Claim 9, **characterized in that** the chromatographic method is an anion-exchange chromatography.

11. Method as defined in Claim 9, **characterized in that** the chromatographic method is an anion-exchange chromatography on an anion-exchange column with grafting of the quaternary ammonium via a covalent bond.

12. Method as defined in Claim 10, **characterized in that** a up to 200 nm transparent mobile phase is used.

13. Method as defined in Claim 12, **characterized in that** the mobile phase is a solution of sodium perchlorate, of methanesulfonate or of phosphate.

14. Method as defined in Claim 13, **characterized in that** the mobile phase is a sodium perchlorate solution.

15. Method as defined in Claim 1, **characterized in that** the method for the selective detection of acetylated sugars is carried out by measuring the absorption at 202-230 nm and 234 nm.

16. Method as defined in Claim 15, **characterized in that** the method for the selective detection of acetylated sugars is carried out by measuring the absorption at 202-230 nm, 234 nm and 280 nm.

17. Use of the method as defined in Claim 1 for the assay of β-unsaturated oligosaccharides.

18. Use of the method as defined in Claim 1 for the assay of enoxaparin, octaparin, bemiparin or tinzaparin.

19. Use of the method as defined in Claim 1 for the assay of one or more of the following octasaccharides:

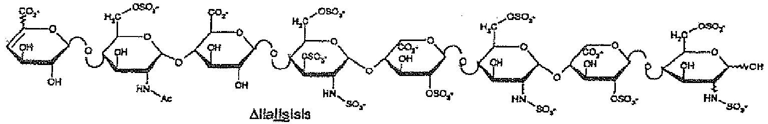

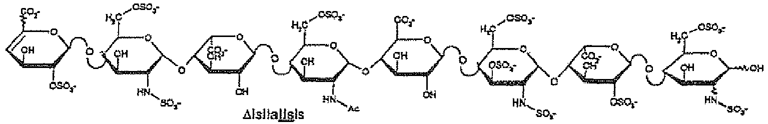

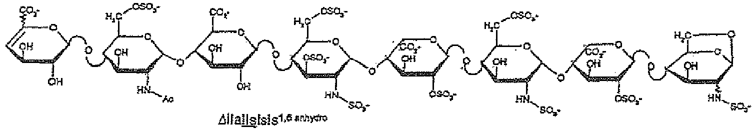

20. Use of the method as defined in Claim 1 for the assay of one or more of the following oligosaccharides:
